(19) Europäisches Patentamt
European Patent Office
Office européen des brevets

(11) **EP 4 557 301 A1**

(12) **EUROPEAN PATENT APPLICATION**

(43) Date of publication:
**21.05.2025 Bulletin 2025/21**

(21) Application number: **23209813.7**

(22) Date of filing: **14.11.2023**

(51) International Patent Classification (IPC):
**G16C 60/00** (2019.01)    **G05B 17/02** (2006.01)

(52) Cooperative Patent Classification (CPC):
**G16C 60/00; G05B 17/02;** G06F 30/20;
G16C 20/30; G16C 20/70

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **MAGMA Gießereitechnologie GmbH
52072 Aachen (DE)**

(72) Inventor: **Weinhold, Georg
52072 Aachen (DE)**

(74) Representative: **Nordic Patent Service A/S
Bredgade 30
1260 Copenhagen K (DK)**

(54) **METHOD FOR PRODUCING THERMO-PHYSICAL MATERIAL DATA FOR POLYMER-BASED PROCESS SIMULATIONS**

(57) A computer-implemented method for improving a simulation (1) of a polymer-based production process (2) by clustering a plurality of known polymers into polymer types, determining a representative parameter (7) for each of a set of thermo-physical properties (4) for each polymer type (6) using a statistical method based on measured parameters (3) of these thermo-physical properties (4), and generating a dataset (8) for an unknown polymer based on a choice of polymer type (6) for the unknown polymer. The generated dataset (8) can be used as input for a simulation (1) of the polymer-based production process (2).

```
┌─────────────────────────────────┐
│      MEASURED PARAMETER         │── 101
└─────────────────────────────────┘
                │
                ▼
┌─────────────────────────────────┐
│        POLYMER TYPES            │── 102
└─────────────────────────────────┘
                │
                ▼
┌─────────────────────────────────┐
│    REPRESENTATIVE PARAMETER     │── 103
└─────────────────────────────────┘
                │
                ▼
┌─────────────────────────────────┐
│           DATASET               │── 104
└─────────────────────────────────┘
                │
                ▼
┌─────────────────────────────────┐
│         SIMULATION              │── 105
└─────────────────────────────────┘
```

FIG. 3

EP 4 557 301 A1

**Description**

TECHNICAL FIELD

The disclosure relates to the field of computer-based simulations of processes in the manufacturing domain, and in particular to improving simulations of injection, compression, transfer molding or extrusion of polymers. The disclosure further relates to a computer-based systems and methods to design, to set up, or to control a production machine involved in the aforementioned processes.

BACKGROUND

Injection, compression or transfer molding are cyclic manufacturing processes characterized by pressing liquid material (especially polymers) into a cavity. In this cavity, surrounded by a mold, the liquid material solidifies, due to e.g. cooling down or vulcanization. During the solidification process the liquid material is transformed into a solid. When the material is solid, it is ejected out of the mold and a new production cycle starts with injecting new material into the empty cavity. The solidified polymer is the produced part. The extrusion process on the other hand is used to create parts of a fixed cross section. In this process the liquid material is continually pressed through a die. The liquid material then solidifies and thus the final shape of the part is fixed.

During the solidification process the material is building up internal stresses, which may lead to a deformation of the manufactured item.

It is desirable to set up and to control the production process (e.g. by controlling production machine parameters, such as temperature, injection speed or pressure) and to design the mold or die in a way that the properties and shape of the parts after all production steps match certain quality criteria (e.g. part shape, surface quality, mechanical or chemical properties).

The correlations of production machine parameters, material behavior and the aforementioned criteria are very complex. It is state of the art to use computer-based simulations in a multiplicity of scenarios, e.g to design a mold including cavity geometry and cooling layout, to find an initial setup of machine parameters, to control an injection molding machine in production or to help to understand why a process is not working as expected.

In all scenarios, all of these correlations are dominated by the used part material and its thermo-physical properties, which are therefore crucial inputs for such computer-based simulations.

Examples of the thermo-physical data needed for a computer-based simulation are thermal conductivity, specific heat, density, viscosity, curing and crystallization kinetics. The collection of all or a subset of needed thermo-physical data is referred to as a dataset.

It is state of the art to measure the needed thermo-physical properties. For each needed thermo-physical property, a specialized experimental setup and adapted interpretation of the experimental data has been developed. For example, the viscosity can be measured by pressing polymer melt at a certain temperature through a die. The pressures needed for a given volume flows are measured. From this pressure and volume flow data, the viscosity as a function of shear rate can be calculated.

Two problems hinder the usage of computer-based simulations of the aforementioned processes.

On the one hand, the deviation of all of thermo-physical quantities with respect to different polymers may be very large. If no concrete information about the thermo-physical quantities can be given, the deviation of a computer-based simulation and the real process behavior may also be very large. This deviation will reduce the benefit of the simulation, since simulated measures to improve the real process are less trustworthy or may even be wrong.

On the other hand, it is very time-consuming and expensive to gain an exact and complete characterization of one material. Due to the large variety of feasible materials, its combination with possible additives (such as fibers, carbon black, coloring or ageing agent) and also the use of recycled material, the exact knowledge about one specific material is often lacking.

Some existing methods attempt to calculate thermo-physical properties based on molecular structure. However, these methods are still not accurate enough, and require specific knowledge of the molecular structure of the polymers in question.

SUMMARY

It is an object to overcome the lack of reliable material data for polymeric materials by providing a method for creating a dataset of acceptable quality, without having to perform the mentioned characterization. Acceptable in this context means that results of a computer-based simulation of a production process using a generated dataset is sufficiently accurate in order to gain a benefit in the mentioned scenarios.

This goal is achieved by a method for generating thermo-physical data for a certain material based on a known property of the material, such as polymer type. While the deviation of some of these thermo-physical properties between polymers in general may be very large, for a specific polymer type the deviation may be significantly smaller than the deviation between all polymers.

According to a first aspect, there is provided a computer-implemented method for improving a simulation of a polymer-based production process, the method including the steps of obtaining measured parameters for a set of thermo-physical properties for each of a plurality of polymers based on physical measurements, clustering the plurality of polymers into polymer types based on at least one clustering criteria, determining a representative parameter for each of the set of thermo-physical properties for each polymer type using a statistical method, and generating a dataset based on a choice of polymer type, the dataset including the determined representative parameters for the chosen polymer type.

In some embodiments, at least one of the plurality of polymers can be clustered into multiple polymer types.

In a possible implementation form of the first aspect, the method includes using the generated dataset as input for a simulation of a polymer-based production process.

In a possible implementation form of the first aspect, at least one clustering criteria may be similarity between molecular structures of the polymers.

In a possible implementation form of the first aspect, at least one clustering criteria may be the presence or amount of added fillers in the polymers. The added fillers may include glass fibers, carbon fibers, calcium carbonate, coloring agents, carbon black, silica and/or oil.

In some embodiments, at least one of the polymer types may be further subdivided based on their atomic structure or the chemical structure of their base polymer.

In some embodiments, the polymer types may include at least one of elastomers, thermoset or thermoplastic materials.

In some embodiments, the measured parameters are 1-dimensional and comprise scalar measured values, such as viscosity, and the determined representative parameters comprise scalar representative values.

In other embodiments, the measured parameters are multidimensional and may comprise a plurality of measured values of a thermo-physical property in relation to certain variables, such as thermal conductivity values measured at different temperatures. In such embodiments the determined representative parameters are corresponding multidimensional representative parameters.

The plurality of measured values may also be interpolated or extrapolated to define e.g. a continuous line or curve (in case of 2-dimensional parameters) or a surface (in case of 3-dimensional parameters). In such cases the determined representative parameters are corresponding lines, curves, or surfaces.

In some embodiments, the statistical method includes calculating a median parameter using the Gaussian distribution of the measured parameters of each of the set of thermo-physical properties in the respective cluster for each polymer type.

In embodiments where the measured parameters are scalar values, the median parameter is a mean scalar value, while in the case of multi-dimensional measured parameters the median parameter is correspondingly multi-dimensional, such as a median curve.

In some embodiments, the set of thermo-physical properties may include thermal conductivity, specific heat, density, viscosity, T90-time, curing or crystallization kinetics, solidification behavior, and/or mechanical properties, such as stiffness, shrinkage, cure shrinkage.

In a possible implementation form of the first aspect, the method further includes the steps of calculating a statistical deviation ($\sigma$) from the representative parameter for at least one of the set of thermo-physical properties using the statistical method, and determining a plurality of alternative representative parameters for the thermo-physical property based on the representative parameter and the calculated statistical deviation ($\sigma$).

In a possible implementation form of the first aspect, a plurality of alternative representative values ($V_{var}$) are calculated by deducting or adding a multiple of the statistical deviation ($\sigma$) from/to a representative value ($V$), i.e. $V_{var} = V +/- (n \times o)$.

In a possible implementation form of the first aspect, the plurality of alternative representative values ($V_{var}$) comprise at least one of a lowest representative value ($V_{min}$), a lower representative value ($V_{low}$), an average representative value ($V_{avg}$), a higher representative value ($V_{high}$), and highest representative value ($V_{max}$).

In an embodiment the lowest representative value ($V_{min}$) is calculated as $V_{min} = V - 2\sigma$.

In an embodiment the lower representative value ($V_{low}$) is calculated as $V_{low} = V - \sigma$.

In an embodiment the average representative value ($V_{avg}$) is given as $V_{avg} = V$.

In an embodiment the higher representative value ($V_{high}$) is calculated as $V_{high} = V + \sigma$.

In an embodiment the highest representative value ($V_{max}$) is calculated as $V_{max} = V + 2\sigma$.

Corresponding methods for calculating lower and higher representative parameters can also be implemented in the case of multi-dimensional representative parameters, such as median curves, where the median curve would be shifted in a positive or negative direction to determine a lower or higher representative curve.

In a possible implementation form of the first aspect, the step of generating a dataset is further based on a choice of a representative parameter from a plurality of alternative representative parameters for at least one thermo-physical property.

In a possible implementation form of the first aspect, the method further includes the steps of defining a set of input parameters based on the choice of polymer type and optionally a choice of a representative parameter for at least one thermo-physical property, and generating a plurality of datasets by varying the input parameters.

In a possible implementation form of the first aspect, the method further includes running a simulation of a production process for each generated dataset, wherein the production process has at least one known output parameter measured in physical reality, and wherein a generated dataset may be used as input thermo-physical material data for a given simulation run to obtain a simulated output parameter of the production process.

In a possible implementation form of the first aspect, the method further includes identifying at least one acceptable dataset that resulted in a simulated output parameter fulfilling at least one quality criteria when compared to the known output parameter.

In a possible implementation form of the first aspect, identifying an acceptable dataset is based on evaluating a plurality of quality criteria defining a multi-dimensional quality threshold with respect to a known output parameter, wherein the acceptable datasets are selected as the datasets resulting in simulated output parameters fulfilling a condition with respect to the quality threshold.

In an embodiment, the condition is that the acceptable datasets are selected as the datasets resulting in simulated output parameters not exceeding the quality threshold.

In an embodiment where the acceptable datasets are based on evaluating two quality criteria, the multi-dimensional quality threshold can be represented by a 2-dimensional line or curve. In other embodiments where the acceptable datasets are based on evaluating more than two quality criteria, the multi-dimensional quality threshold can be represented by a surface expressed in 3 or more dimensions.

In a possible implementation form of the first aspect, at least one of the thermo-physical properties is a multi-parameter thermo-physical property, such as curing degree over time at a given temperature, and the method further comprises the steps of:

- obtaining a plurality of measured values for the multi-parameter thermo-physical property based on physical measurements of a plurality of polymers; and
- determining a master curve or a constitutive equation that defines a master curve for the multi-parameter thermo-physical property best approximating a plurality of measured values for a given polymer type;

wherein for generating a dataset, the representative parameters of the multi-parameter thermo-physical property for a chosen polymer type are determined using the master curve or its constitutive equation.

In a possible implementation form of the first aspect, the constitutive equation is a combination of a mathematical expression and associated parameters, and the method further comprises optimizing the associated parameters by

- calculating errors as differences between the plurality of measured values and corresponding calculated values given by the constitutive equation;

- calculating an error measure,; and
- determining a set of associated parameters that result in a minimum of the error measure.

In an embodiment the error measure is the sum of all squared errors.

In a possible implementation form of the first aspect, the method further comprises the steps of:

- obtaining an input value for a multi-parameter thermo-physical property of a chosen polymer type;
- selecting a master curve or a constitutive equation that defines a master curve for the multi-parameter thermo-physical property based on the chosen polymer type; and
- adjusting the master curve or the constitutive equation to generate a manipulated master curve that matches with the obtained input value.

In a possible implementation form of the first aspect, for generating a dataset, the representative values of a multi-parameter thermo-physical property for a chosen polymer type are determined using a manipulated master curve or adjusted constitutive equation.

In a possible implementation form of the first aspect, generating a manipulated master curve comprises stretching a master curve in a direction or applying an offset until the obtained input value fits on the master curve.

In another possible implementation form of the first aspect, generating a manipulated master curve comprises inverse fitting associated parameters of the corresponding constitutive equation defining the master curve until the obtained input value fits on the master curve.

In a possible implementation form of the first aspect, the method further includes using a result of the simulation of a polymer-based production process to set up or to control a production machine involved in the polymer-based production process, such as an injection molding machine.

In some embodiments, the simulation result may be used for controlling production machine parameters, such as temperature, injection speed or pressure.

In a possible implementation form of the first aspect, the method further includes using a result of the simulation of a polymer-based production process in the process of designing a part to be used in the production process, such as a mold for an injection molding process or a die for an extrusion process.

In some embodiments, the simulation result may be used to design part shape, surface quality, mechanical or chemical properties of a mold or die.

According to a second aspect, there is provided a computer-based system comprising an input-output interface; a display; a non-transitory machine-readable storage medium including a computer program product;

and at least one processor operable to execute the program product, interact with the input-output interface and the display, and perform operations according to the methods of any one of the possible implementation forms of the first aspect.

According to a third aspect, there is provided a computer program product, encoded on a non-transitory machine-readable storage medium, operable to cause a processor to perform operations according to the methods of any one of the possible implementation forms of the first aspect.

These and other aspects will be apparent from the embodiment(s) described below.

BRIEF DESCRIPTION OF THE DRAWINGS

In the following detailed portion of the present disclosure, the aspects, embodiments and implementations will be explained in more detail with reference to the example embodiments shown in the drawings, in which:

Fig. 1 illustrates a possible way to cluster polymers into polymer types according to an embodiment of the disclosure.

Fig. 2 illustrates the deviation of values of a given thermo-physical property within all polymer types.

Fig. 3 is a flow diagram illustrating a method for improving a simulation of a polymer-based production process according to an embodiment of the disclosure.

Fig. 4 illustrates a method of clustering polymers into polymer types according to an embodiment of the disclosure.

Fig. 5 illustrates a method of a determining a representative value of a thermo-physical property for a given polymer type according to an embodiment of the disclosure.

Fig. 6 is a flow diagram illustrating a method for generating an enhanced dataset for a polymer-based process simulation according to an embodiment of the disclosure.

Fig. 7 illustrates a set of input parameters based on a choice of polymer type and choices of representative values for thermo-physical properties according to an embodiment of the disclosure.

Fig. 8 is a flow diagram illustrating a method for determining an acceptable dataset by running a plurality of simulations according to an embodiment of the disclosure.

Fig. 9 is a diagram illustrating a selection of acceptable datasets based on multiple quality criteria according to an embodiment of the disclosure.

Fig. 10 is a diagram illustrating the step of determining a master curve for a multi-parameter thermo-physical property for a given polymer type according to an embodiment of the disclosure.

Figs. 11 and 12 are diagrams illustrating the steps of manipulating a master curve to match a given input value according to an embodiment of the disclosure.

Fig. 13 is a flow diagram illustrating a method for generating a dataset for a polymer-based process simulation using a given input value for a multi-parameter thermo-physical property according to an embodiment of the disclosure.

Fig. 14 is a flow diagram illustrating a method for setting up or controlling a production machine, or designing a part to be used in a polymer-based production process according to embodiments of the disclosure.

Fig. 15 is a block diagram illustrating a computer-based system according to a second aspect of the disclosure.

DETAILED DESCRIPTION OF THE FIGURES

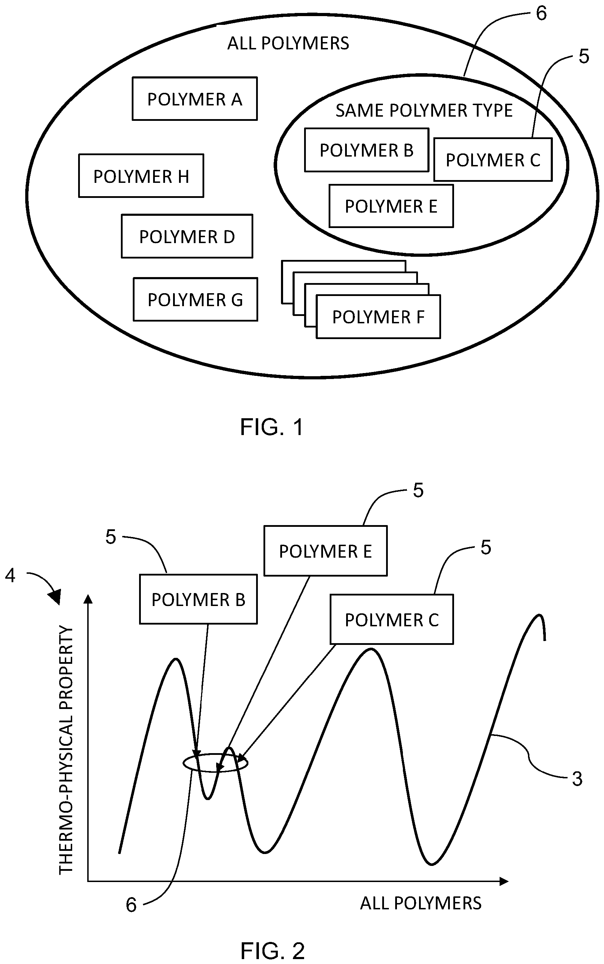

Fig. 1 illustrates a possible way to cluster polymers 5 into polymer types 6 according to an embodiment of the disclosure. Polymeric materials or polymers 5 (represented in Fig. 1 in a simplified manner by letters ranging from A to H) can be clustered on different levels based on different clustering criteria. On a coarse level one can distinguish e.g. elastomers, thermoset and thermoplastic materials. Each of these polymers 5 can also be characterized by the chemical structure of their base polymer. Elastomers for example can be subdivided by their atomic structure (heteroatom). Examples for these subdivisions are natural rubber, polybutadiene, ethylene propylene rubber. The presence or amount of added fillers may also be used as a subdivision. Examples for fillers are glass fibers, carbon fibers, calcium carbonate, coloring agents, carbon black, silica and/or oil. These subdivisions will be referred to in the disclosure as polymer types 6 or polymer type 6 in singular form.

Knowledge of certain thermo-physical properties 4 of these polymers 5 are essential for executing computer-based simulations of production processes 2 that involve use of such polymers 5, for example to design a mold including cavity geometry and cooling layout, to find an initial setup of machine parameters, to control an injection molding machine in production or to help to understand why a process is not working as expected. Examples of the thermo-physical data needed for a computer-based simulation are thermal conductivity, specific heat, density, viscosity, T90-time, curing or crystallization kinetics, solidification behavior, and/or mechanical properties, such as stiffness, shrinkage, cure shrinkage. The collection of all or a subset of needed data on thermo-physical properties 4 is referred to as a dataset 8.

For each needed thermo-physical property 4, specialized experimental setups and adapted interpretations of the experimental data have been developed to gather measured values 3A into such datasets 8 stored in a database.

When launching a simulation, users usually select a dataset 8 from a database based on one specific value, such as an expected value or measured value of the starting material. However, if no concrete information about the thermo-physical properties 4 of a certain polymer 5 can be given as input, the deviation of a computer-based simulation and the real process behavior may be very large. This deviation will reduce the benefit of the simulation, since simulated measures to improve the real process are less trustworthy or may even be wrong.

This problem occurs frequently with polymer-based simulations since it is very time-consuming and expensive to gain an exact and complete characterization of a specific polymer to be used. Due to the large variety of feasible materials, its combination with possible additives, such as fibers, carbon black, coloring or ageing agent, and also the use of recycled material, the exact knowledge about one specific polymer is therefore often lacking.

The disclosed method intends to drastically improve the simulation quality in such scenarios where there is no existing dataset for the exact polymer 5 intended to be used in the simulated production process, and the user needs to select a dataset that they believe matches best the properties of the polymer 5 to be used.

Fig. 2 shows the large deviations of measured values 3 of a certain thermo-physical property 4 for a plurality of polymers 5 on a diagram. As also shown in Fig. 2, for a selected cluster of these polymers 5 of the same polymer type 6, these deviations between measured values 3 are much smaller. This gave the inventive idea for the inventors for using a method based on clustering polymers 5 into polymer types 6 for generating datasets 8 for computer-based simulations 1, as will be explained below.

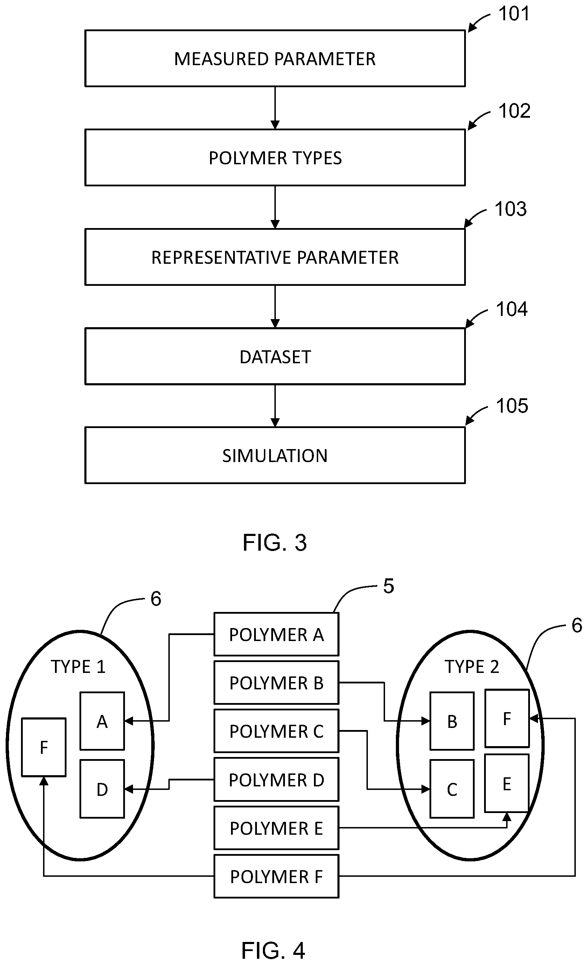

Fig. 3 is a flow diagram illustrating a method for improving a simulation 11 of a polymer-based production process according to an embodiment of the disclosure.

In a first step 101, measured parameters 3 are obtained for a set of thermo-physical properties 4 for each of a plurality of known polymers 5, using state of the art testing procedures.

In a next step 102, the plurality of polymers 5 are clustered into polymer types 6 based on at least one clustering criteria, as shown in Fig. 1. This clustering criteria may be the molecule structure, filler ingredients, or any other criteria as described before.

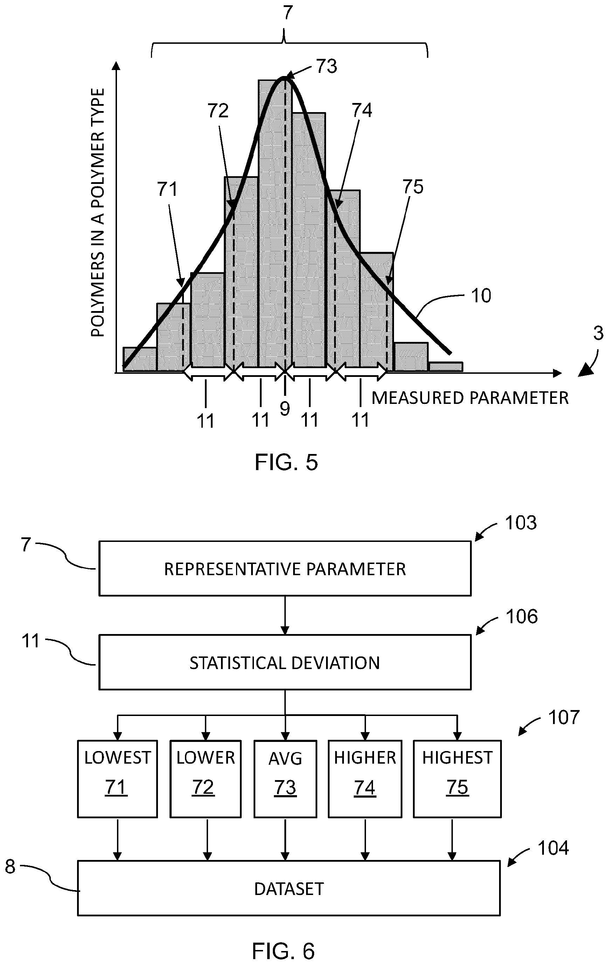

In a next step 103, for all polymer types 6, statistical methods are applied to identify one representative parameter 7 for each thermos-physical property 4. This may be performed by calculating the mean value 9 of the Gaussian distribution 10 of measured values 3A of a given thermo-physical property 4, as illustrated in Fig. 5 and described in more detail below.

Once polymer types 6 are identified, and the above representative parameters 7 are calculated, a dataset 8 can be generated based on a choice of polymer type 6 in a next step 104. This dataset 8 will comprise the determined representative parameters 7 for the chosen polymer type 6. In a final step 105, the generated dataset 8 can be used as input for a simulation 1 of a polymer-based production process 2.

Fig. 4 illustrates a method of clustering polymers 5 into polymer types 6 according to an embodiment of the disclosure. As shown in this figure, certain polymers 5 may be clustered into only one polymer type 6 based on the clustering criteria as described before, while other polymers 5 (shown in the figure as Polymer F) may be clustered into more than one polymer type 6.

Figs. 5 and 6 illustrate a statistical method for determining representative values 7 of a thermo-physical property 4 for a given polymer type 6 according to an embodiment of the disclosure, to be used for generating an enhanced dataset 8 for a simulation 1 of a polymer-based production process 2. In the illustrated embodiment as shown in Fig. 5, the statistical method comprises calculating the mean value 9 of the Gaussian distribution 10 of measured scalar values 3A for a given thermo-physical property 4. This mean value 9 can then be used as a sole representative parameter 7, or as one of the representative parameters 7 to choose from when generating a dataset 8, as shown in the flowchart of Fig. 6, with the results of the steps shown in Fig. 6 illustrated on the diagram of Fig. 5.

In particular, in the latter mentioned case, the statistical method further comprises, after determining a representative parameter 7, a next step 106 of calculating a statistical deviation 11 from the representative parameter 7 for a chosen thermo-physical property 4.

In a following step 107, based on the representative parameter 7 and the calculated statistical deviation 11, a plurality of alternative representative parameters 71, 72, 73, 74, 75 can be calculated for the chosen thermo-physical property 4. The plurality of alternative representative parameters 71, 72, 73, 74, 75 can be calculated by deducting or adding multiples of the statistical deviation 11 from/to the representative parameter 7. This can result in a selection of five alternative representative parameters, such as: a lowest representative parameter 71, lower representative parameter 72, average representative parameter 73, higher representative parameter 74, and highest representative parameter 73, as illustrated in Fig. 5.

According to an embodiment, where the representative parameters are scalar values, alternative representative values can be calculated as follows:

- the lowest representative value ($V_{min}$) is calculated as

$$V_{min} = V - 2\sigma;$$

- the lower representative value ($V_{low}$) is calculated as

$$V_{low} = V - \sigma;$$

- the average representative value ($V_{avg}$) is given as

$V_{avg} = V$;

- the higher representative value ($V_{high}$) is calculated as

$$V_{high} = V + \sigma;$$

and

- the highest representative value ($V_{max}$) is calculated as

$$V_{max} = V + 2\sigma.$$

The step 104 of generating a dataset 8, as illustrated in Fig. 3 and described before, is then based on one or multiple choices between alternative representative parameters 71, 72, 73, 74, 75 for at least one thermo-physical property 4.

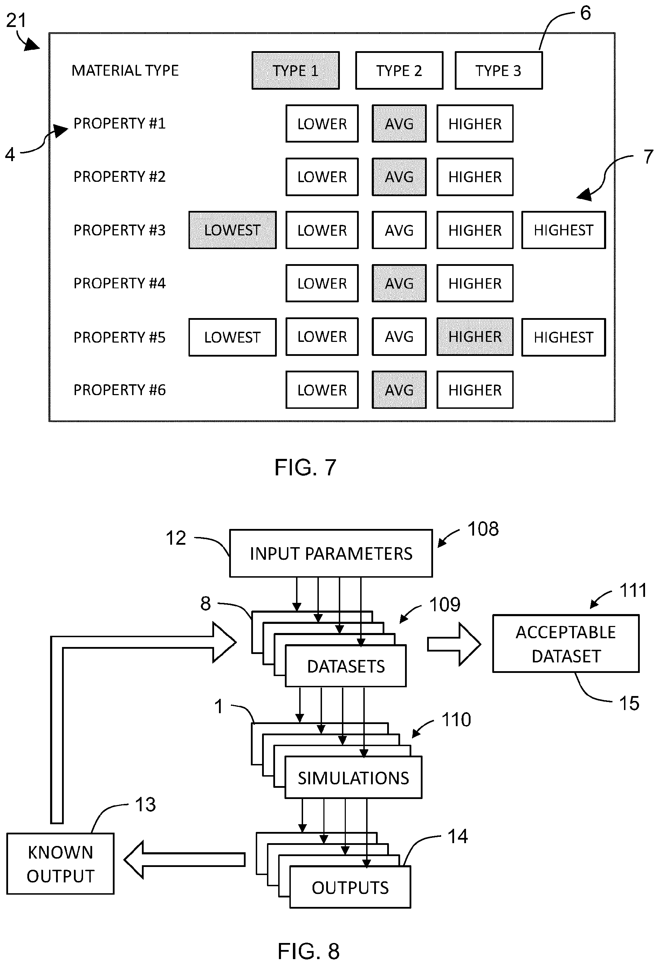

This choice is further illustrated in Fig. 7, where a choice between polymer types 6 and choices of representative parameters 7 for different thermo-physical properties 4 are given by a user through a graphical user interface (GUI) 21, as a set of input parameters 12, for generating a dataset 8 to be used as input in a simulation 1 of a polymer-based production process 2.

This embodiment is especially useful if for a particular dataset 8 a tendency of thermo-physical property 4, in comparison to other polymer types 6, is known. For example, using a Gaussian distribution: if it is known that the conductivity is particularly low in comparison to other polymers 5 of the same polymer type 6, the mean value 9 minus the statistical deviation 11 of the conductivity may be used. It is then possible to generate multiple datasets 8 by varying the choices between alternative representative parameters 7. One may then simulate already well understood production processes with this variety of datasets 8. Then one can identify which dataset 8 has performed best, as illustrated in more detail in Fig. 8.

Fig. 8 illustrates in a flow diagram a method for determining an acceptable dataset 15 by running a plurality of simulations 1 based on a varying set of input parameters 12, which can be given through a GUI as illustrated in Fig. 7.

In a first step 108 a set of input parameters 12 are defined so that multiple datasets 8 can be generated. The input parameters 12 are based on the choice of polymer type 6 and a choice of a representative parameter 7 for at least one thermo-physical property 4. The number of these alternative parameters can vary between thermo-physical properties 4 based on known trends regarding a given thermo-physical property 4. For some thermo-physical properties 4 only one representative parameter 7 may be given, for others a lower 72 and a higher 74 parameter may be given as alternative choices as well as an average 73 parameter, and for others a further lowest 71 and highest 75 parameter may be given as alternative options to choose from.

Then in a next step 109 a plurality of datasets 8 are generated by varying these input parameters 12.

In a following step 110 a simulation 1 of a production process 2 is executed for each generated dataset 8, where each generated dataset 8 is used as input thermo-physical material data for a given simulation 1 to result in a simulated output parameter 14 of the production process 2.

It is an important condition for this method that the production process 2 has at least one known output parameter 13 measured in physical reality.

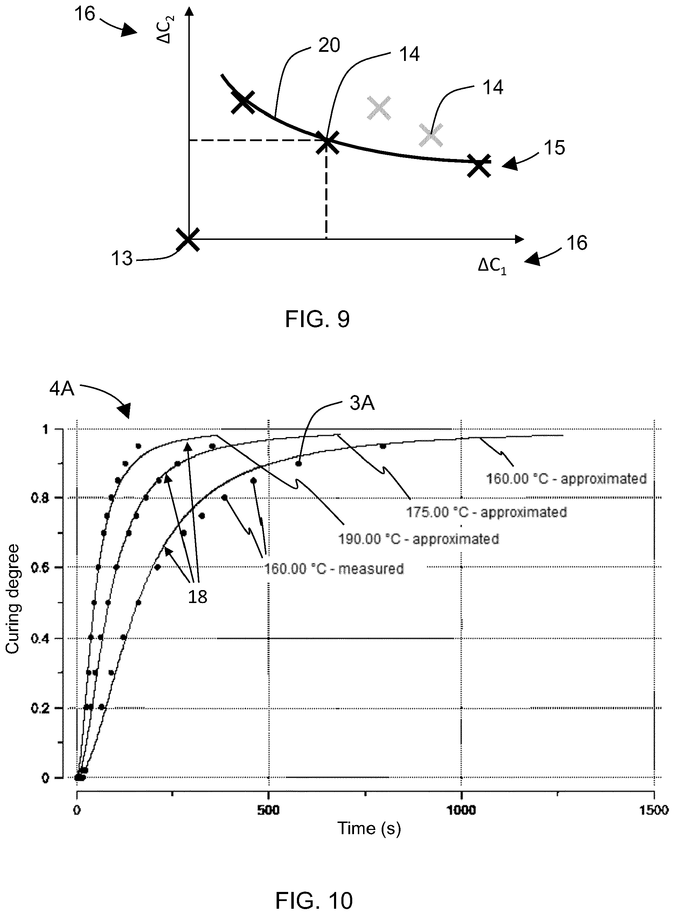

This way, in a final step 111 at least one acceptable dataset 15 can be identified, based on which simulation 1 resulted in a simulated output parameter 14 fulfilling a quality criterion 16 when compared to the known output parameter 13. This step may be based on only one quality criterion 16, such as a deviation of a simulated output parameter 14 from the known output parameter 13 being below a given threshold, or may be determined based on evaluating multiple quality criteria 16, as further illustrated in Fig. 9.

Fig. 9 is a diagram illustrating a selection of acceptable datasets 15 based on multiple quality criteria 16 according to an embodiment of the disclosure. In this illustrated embodiment, identifying acceptable dataset(s) 15 is based on a plurality of quality criteria 16 defining a multi-dimensional quality threshold 20 with respect to the known output parameter 13, wherein the acceptable datasets 15 are selected as the datasets 8 resulting in simulated output parameters 14 fulfilling a condition with respect to the quality threshold 20.

In an embodiment, the condition is that the acceptable datasets 15 are selected as the datasets 8 resulting in simulated output parameters 14 not exceeding the quality threshold 20.

In an embodiment, as illustrated in Fig. 9, where the acceptable datasets 15 are based on evaluating two quality criteria 16, the multi-dimensional quality threshold 20 is represented by a 2-dimensional line or curve. Each of the simulated output parameters 14 fulfilling the quality threshold criteria may represent acceptable datasets 15, with no distinction between quality of these acceptable datasets 15. The sum of acceptable datasets 15 represents the pareto set, which is indicated by the simulated output parameters 14 located on the curve 20 in figure 9.

In other embodiments where the acceptable datasets 15 are based on evaluating more than two quality criteria 16, the multi-dimensional quality threshold 20 can also be represented by a surface expressed in 3 or more dimensions.

There may also be cases where a given thermo-physical property 4 to be used for generating datasets 8 is a multi-parameter thermo-physical property 4A, such as curing degree over time at a given temperature. Figs. 10-13 illustrate steps of a method in such cases involving at least one multi-parameter thermo-physical property 4A in the given set of thermo-physical properties 4.

Fig. 10 is a diagram illustrating the step of determining a master curve 18 for a multi-parameter thermo-physical property 4A for a given polymer type 6 according to an embodiment of the disclosure.

In this case, the method comprises a first step 101A of obtaining a plurality of measured values 3A for the multi-parameter thermo-physical property 4A, i.e. the curing degree over time at a given temperature, based on physical measurements of a plurality of polymers 5. In the illustrated example of Fig. 10, measured values 3A are obtained for curing degrees between 0 and 1 (or 0 and 100%) over time period of 0 to 1000 s, for temperatures of 160 °C, 175 °C, and 190 °C. Based on these obtained measured values 3A, in a next step 112 a master curve 18 - or a constitutive equation that defines a master curve 18 - is determined for the multi-parameter thermo-physical property 4A, based on a best approximation of the plurality of measured values 3A.

The dataset 8 for a chosen polymer type 6 can then be generated by determining representative parameters 7 of this multi-parameter thermo-physical property 4A using the master curve 18 or its constitutive equation.

This constitutive equation is a combination of a mathematical expression and associated parameters, which associated parameters can be further optimized. In such cases, the method further comprises calculating errors as differences between the plurality of measured values 3A and corresponding calculated values given by the constitutive equation.

Then, an error measure, such as the sum of all squared errors is defined, and a set of optimized associated parameters are calculated as the associated parameters that result in a minimum of this error measure.

In certain cases, where a given input value 17 for a multi-parameter thermo-physical property 4A is known, the master curve 18 or its constitutive equation can be adjusted, in order to generate a dataset better matching the given input value 17.

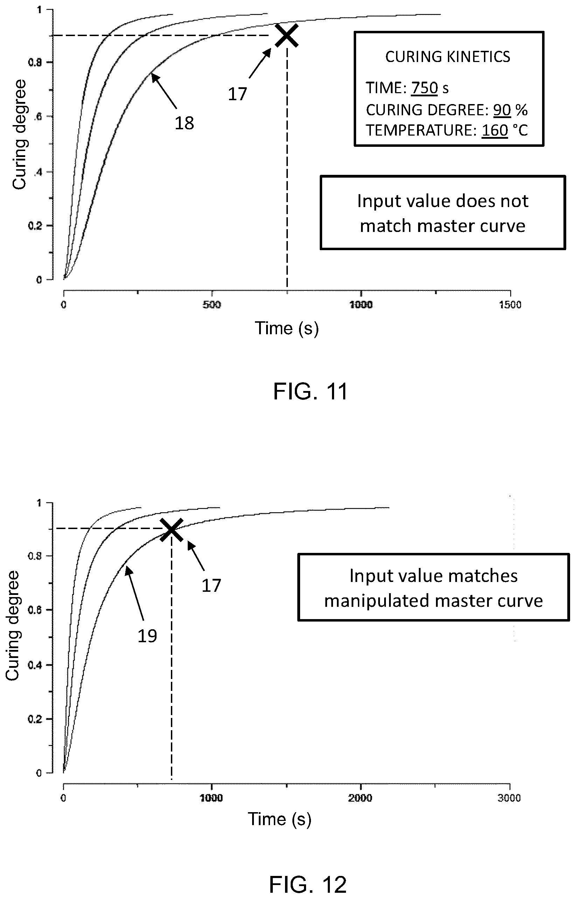

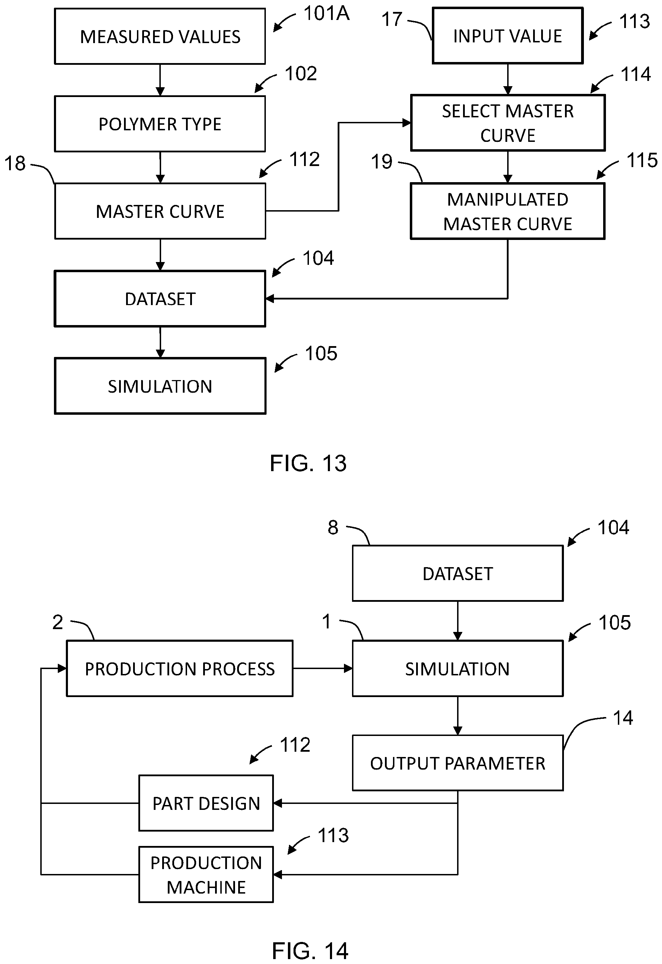

Figs. 11 and 12 are consecutive diagrams, while Fig. 13 is a flow chart illustrating the steps of manipulating a master curve 18 to match a given input value 17 according to an embodiment of the disclosure.

As shown in Fig. 11 and the flowchart of Fig. 13, in a first step 113 an input value 17 for a multi-parameter thermo-physical property 4A of a chosen polymer type 6 is obtained. As an example, this input value 17 defines curing kinetics as shown in the above example of Fig. 10, in this particular example the input value 17 is a curing degree of 90% or 0.9, at a time of 750 s, at a given temperature of 160 °C.

In a next step 114, a master curve 18 (or a constitutive equation that defines the master curve 18) for the multi-parameter thermo-physical property 4A is selected based on the chosen polymer type 6 - in this case the master curve 18 for the given temperature of 160 °C. As can be seen in Fig. 11, the input value 17 does not match this master curve 18.

Fig. 12 shows the next steps of manipulating the master curve 18 to fit the input value 17, as also explained in the flowchart of Fig. 13.

In a next step 115, the master curve 18 (or the constitutive equation) is adjusted to generate a manipulated master curve 19 that matches with the obtained input value 17. Generating the manipulated master curve 19 may comprise different methods, as simple as stretching the master curve 18 in a direction or applying an offset until the obtained input value 17 fits on the generated manipulated master curve 19, or even inverse fitting associated parameters of the constitutive equation until the obtained input value 17 fits on the generated manipulated master curve 19.

As shown in Fig. 13, once the manipulated master curve 19 is generated, it can be used for generating the dataset 8, wherein the representative parameters 7 of the multi-parameter thermo-physical property 4A for a chosen polymer type 6 are determined using the manipulated master curve 19 (or its adjusted constitutive equation).

Fig. 14 is a flow diagram illustrating computer-based methods for setting up or controlling a production machine 22, or designing a part to be used in a polymer-based production process 2 according to embodiments of the disclosure.

As illustrated in the flow diagram, the aforementioned methods may further include, following the step 104 of generating a dataset and step 105 of running a simulation 105 of polymer-based production process 2, a step 112 of setting up or controlling a production machine 22 involved in the polymer-based production process 2, such as an injection molding machine, using a simulated output parameter 14 of the simulation 1 of the polymer-based production process 2.

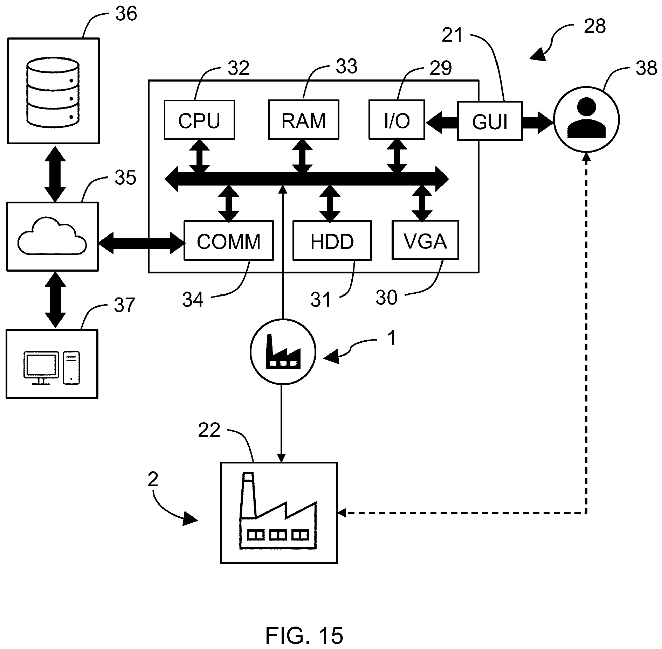

In some embodiments, the simulation results 14 may be used for controlling parameters of the production machine 22, such as temperature, injection speed or pressure, as illustrated in Fig. 15.

As further illustrated in the flow diagram of Fig. 14, the aforementioned methods may also include, following the step 104 of generating a dataset and step 105 of running a simulation 1 of polymer-based production process 2, a step 113 of designing a part to be used in the production process 2, such as a mold for an injection molding process or a die for an extrusion process, using a simulated output parameter 14 of the simulation 1 of the polymer-based production process 2.

In some embodiments, the simulation result 14 may be used to design part shape, surface quality, mechanical or chemical properties of a mold or die.

Fig. 15 is a schematic block diagram illustrating an example of a hardware configuration of a computer-based system 28 for running a simulation 1 of polymer-based production process 2 and optionally for setting up or controlling a production machine 22 involved in the polymer-based production process 2 in accordance with the present disclosure.

The computer-based system 28 may comprise one or more processors (CPU) 32 configured to execute instructions that cause the computer-based system to perform a method according to any of the possible embodiments described above.

The computer-based system 28 may also comprise computer-readable storage medium 31 configured for storing software-based instructions as part of a program product to be executed by the CPU 32.

The computer-based system 28 may also comprise a memory 33 configured for (temporarily) storing data of applications and processes.

The computer-based system 28 may further comprise an input-output interface 29 for user interaction between the system and a user 38, connected to or comprising an input interface (such as a keyboard and/or mouse) for receiving input from a user 38, and an output device such as an electronic display 30 for conveying information to a user 38 via a graphical user interface (GUI) 21, such as the GUI 21 illustrated in Fig. 7.

The computer-based system 28 may further comprise a communications interface 34 for communicating with external devices such as a remote client 37 directly, or indirectly via a computer network 35.

The mentioned hardware elements within the computer-based system may be connected via an internal bus configured for handling data communication and processing operations.

The computer-based system 28 may further be connected to a database 36 configured for storing data to be used as input for the above-described methods (such as measured parameters 3 of thermo-physical properties 4 for various polymers 5, or sets of measured values 3A for multi-parameter thermo-physical properties 4A), wherein the type of connection between the two can be direct or indirect. The computer-based system 28 and the database 36 can be both included in the same physical device, connected via the internal bus, or they can be part of physically different devices, and connected via the communication interface 34 either directly, or indirectly via a computer network 35.

As mentioned before, the computer-based system 28 may further be connected to a production machine 22 of a polymer-based production process 2, and simulation results 14 generated by the computer-based system 28 may be used for controlling parameters of the production machine 22, such as temperature, injection speed or pressure. Parameters can also be obtained from the production machine 22 for improving the simulations 1 and/or the datasets 8 used for the simulations 1, as described before in relation to Fig. 8.

The various aspects and implementations have been described in conjunction with various embodiments herein. However, other variations to the disclosed embodiments can be understood and effected by those skilled in the art in practicing the claimed subject-matter, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems.

The reference signs used in the claims shall not be construed as limiting the scope.

## Claims

1. A computer-implemented method for improving a simulation (1) of a polymer-based production process (2), the method comprising the steps of:

   (101) obtaining measured parameters (3) for a set of thermo-physical properties (4) for each of a plurality of polymers (5) based on physical measurements;
   (102) clustering said plurality of polymers (5) into polymer types (6) based on at least one clustering criteria;
   (103) determining a representative parameter (7) for each of said set of thermo-physical properties (4) for each polymer type (6) by applying a statistical method to the measured parameters (3) of a given thermo-physical property (4) for a given polymer type (6);
   (104) generating a dataset (8) based on a choice of polymer type (6), said dataset (8) comprising said determined representative parameter (7) for the chosen polymer type (6); and
   (105) using the generated dataset (8) as input for a simulation (1) of a polymer-based production process (2).

2. The method of claim 1, wherein at least one clustering criteria is similarity between molecular structures of the polymers (5).

3. The method of any one of claims 1 or 2, wherein at least one clustering criteria is the presence or amount of added fillers in the polymers (5).

4. The method of any one of claims 1 to 3, wherein at least one of said polymer types (6) is further subdivided based on their atomic structure or the chemical structure of their base polymer (5).

5. The method of any one of claims 1 to 4, wherein said statistical method comprises calculating a median parameter (9) using the Gaussian distribution (10) of the measured parameters (3) of each of said set of thermo-physical properties (4) in the respective cluster for each polymer type (6).

6. The method of any one of claims 1 to 5, further comprising the steps of:

(106) calculating a statistical deviation (11) from the representative parameter (7) for at least one of said set of thermo-physical properties (4) using a statistical method, and
(107) determining a plurality of alternative representative parameters (71, 72, 73, 74, 75) for said at least one thermo-physical property (4) based on said representative parameter (7) and the calculated statistical deviation (11), wherein said step (104) of generating a dataset (8) is further based on a choice of a representative parameter (7) from said plurality of alternative representative parameters (71, 72, 73, 74, 75) for at least one thermo-physical property (4).

7. The method of any one of claims 1 to 6, further comprising the steps of:

(108) defining a set of input parameters (12) based on said choice of polymer type (6) and optionally a choice of a representative parameter (7) for at least one thermo-physical property (4);
(109) generating a plurality of datasets (8) by varying said input parameters (12);
(110) running a simulation (1) of a production process (2) for each generated dataset (8), wherein said production process (2) has at least one known output parameter (13) measured in physical reality, and wherein a generated dataset (8) is used as input thermo-physical material data for a given simulation (1) run to obtain a simulated output parameter (14) of said production process (2); and
(111) identifying at least one acceptable dataset (15) that resulted in a simulated output parameter (14) fulfilling a quality criterion (16) when compared to said known output parameter (13).

8. The method of claim 7, wherein identifying said at least one acceptable dataset (15) is based on a plurality of quality criteria (16) defining a multi-dimensional quality threshold (20) with respect to said known output parameter (13), wherein the acceptable datasets (15) are selected as the datasets (8) resulting in simulated output parameters (14) fulfilling a condition with respect to said quality threshold (20).

9. The method of any one of claims 1 to 8, wherein at least one of said set of thermo-physical properties (4) is a multi-parameter thermo-physical property (4A), such as curing degree over time at a given temperature, and wherein the method further comprises the steps of:

(101A) obtaining a plurality of measured values (3A) for said multi-parameter thermo-physical property (4A) based on physical measurements of a plurality of polymers (5); and
(112) determining a master curve (18) or a constitutive equation that defines a master curve (18) for said multi-parameter thermo-physical property (4A) best approximating a plurality of measured values (3A) for a given polymer type; wherein for (104) generating said dataset (8) the representative parameters (7) of said multi-parameter thermo-physical property (4A) for a chosen polymer type (6) are determined using said master curve (18) or constitutive equation.

10. The method of claim 9, wherein said constitutive equation is a combination of a mathematical expression and associated parameters, and wherein the method further comprises optimizing the associated parameters by

- calculating errors as differences between the plurality of measured values (3A) and corresponding calculated values given by the constitutive equation;
- calculating an error measure, such as the sum of all squared errors; and
- determining a set of associated parameters that result in a minimum of said error measure.

11. The method of any one of claims 9 or 10, further comprising the steps of:

(113) obtaining an input value (17) for a multi-parameter thermo-physical property (4A) of a chosen polymer type (6);
(114) selecting a master curve (18) or a constitutive equation that defines a master curve (18) for said multi-parameter thermo-physical property (4A) based on the chosen polymer type (6); and
(115) adjusting said master curve (18) or said constitutive equation to generate a manipulated master curve (19) that matches with the obtained input value (17);
wherein for (104) generating said dataset (8) the representative values (7) of said multi-parameter thermo-physical property (4A) for a chosen polymer type (6) are determined using said manipulated master curve (19) or adjusted constitutive equation.

12. The method of any one of claims 1 to 11, further comprising the step of (112) setting up or controlling a production machine (22) involved in said polymer-based production process (2), such as an injection molding machine, using a simulated output parameter (14) of said simulation (1) of a polymer-based production process (2).

13. The method of any one of claims 1 to 12, further comprising the step of (113) designing a part to be used in said production process (2), such as a mold for an injection molding process or a die for an extrusion process, using a simulated output parameter (14) of said simulation (1) of a polymer-based production process (2).

14. A computer-based system comprising:

> an input-output interface (29);
> a display (30);
> a non-transitory machine-readable storage medium (31) including a computer program product; and
> at least one processor (32) operable to execute the program product, interact with the input-output interface (29) and the display (30), and perform operations according to the methods of any one of the claims 1 to 13.

15. A computer program product, encoded on a non-transitory machine-readable storage medium (31), operable to cause a processor (32) to perform operations according to the methods of any one of claims 1 to 13.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

**EP 4 557 301 A1**

Europäisches
Patentamt

European
Patent Office

Office européen
des brevets

# EUROPEAN SEARCH REPORT

Application Number

EP 23 20 9813

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CASSOLA STEFANO ET AL: "Machine learning for polymer composites process simulation – a review", COMPOSITES PART B, vol. 246, 1 November 2022 (2022-11-01), page 110208, XP093147777, AMSTERDAM, NL ISSN: 1359-8368, DOI: 10.1016/j.compositesb.2022.110208 * the whole document * | 1-15 | INV. G16C60/00 G05B17/02 |
| X | SINGH PARAMJOT ET AL: "Estimating Powder-Polymer Material Properties Used in Design for Metal Fused Filament Fabrication (DfMF)", JOM: JOURNAL OF METALS, SPRINGER NEW YORK LLC, UNITED STATES, vol. 72, no. 1, 12 November 2019 (2019-11-12), pages 485-495, XP036968344, ISSN: 1047-4838, DOI: 10.1007/S11837-019-03920-Y [retrieved on 2019-11-12] * the whole document * | 1-15 | |
| A | K.W JOHNSON: "Grouping materials and processes for the designer: an application of cluster analysis", MATERIALS AND DESIGN, vol. 23, no. 1, 4 December 2001 (2001-12-04), pages 1-10, XP093157809, GB ISSN: 0261-3069, DOI: 10.1016/S0261-3069(01)00035-8 * the whole document * | 1-15 | |

| | TECHNICAL FIELDS SEARCHED (IPC) |
|---|---|
| | G16C G06F G05B |

The present search report has been drawn up for all claims

| Place of search | Date of completion of the search | Examiner |
|---|---|---|
| The Hague | 2 May 2024 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

20